(19) Europäisches Patentamt
European Patent Office
Office européen des brevets

(11) Veröffentlichungsnummer: **0 518 153 A1**

(12) # EUROPÄISCHE PATENTANMELDUNG

(21) Anmeldenummer: **92109193.0**

(51) Int. Cl.⁵: **C07D 263/20**, A01N 43/76

(22) Anmeldetag: **01.06.92**

(30) Priorität: **14.06.91 DE 4119611**

(43) Veröffentlichungstag der Anmeldung:
**16.12.92 Patentblatt 92/51**

(84) Benannte Vertragsstaaten:
**BE CH DE DK FR GB IT LI NL**

(71) Anmelder: **BAYER AG**
**W-5090 Leverkusen 1 Bayerwerk(DE)**

(72) Erfinder: **Elbe, Hans-Ludwig, Dr.**
**Dasnöckel 59**
**W-5600 Wuppertal 11(DE)**
Erfinder: **Böhm, Stefan, Dr.**
**Andreas-Gryphius Strasse 9**
**W-5000 Köln 80(DE)**
Erfinder: **Berg, Dieter, Prof. Dr.**
**Gellertweg 27**
**W-5600 Wuppertal 1(DE)**
Erfinder: **Dehne, Heinz-Wilhelm, Dr.**
**Krischer Strasse 81**
**W-4019 Monheim(DE)**
Erfinder: **Dutzmann, Stefan**
**Kosenberg 10**
**W-4010 Hilden 1(DE)**

(54) **Mittel zur Bekämpfung von Schädlingen auf Basis substituierter Oxazolidinone, neue Substituierte Oxazolidinone, deren Herstellung und Verwendung.**

(57) Teilweise bekannte substituierte Oxazolidinone der Formel (I)

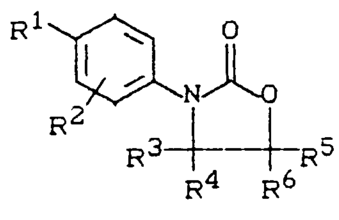

(I)

in welcher
$R^1$ bis $R^6$ die in der Beschreibung gegebenen Bedeutungen haben, zur Verwendung zur Bekämpfung von Schädlingen.

Die neuen und bekannten Verbindungen können nach Analogieverfahren hergestellt werden, indem man geeignete substituierte Isocyanate mit geeigneten Oxiranen umsetzt oder geeignete Oxazolidinone oxidiert.

EP 0 518 153 A1

Die Erfindung betrifft die Verwendung von teilweise bekannten substituierten Oxazolidinonen sowie neue substituierte Oxazolidinone, mehrere Verfahren zu ihrer Herstellung und ihre Verwendung als Schädlingsbekämpfungsmittel.

Es ist bekannt, daß bestimmte substituierte Oxazolidinone wie beispielsweise die Verbindung 5-Fluormethyl-3-(4-methylthiophenyl)-1,3-oxazolidin-2-on fungizide Eigenschaften besitzen (vergleiche z. B. US 4.128.654).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin sind bestimmte substituierte Oxazolidinone, wie beispielsweise die Verbindung 5-Chlormethyl-3-(4-trifluormethylthiophenyl)-1,3-oxazolidin-2-on oder die Verbindung 5-Chlormethyl-3-(4-trifluormethylsulfonylphenyl)-1,3-oxazolidin-2-on bekannt (vergleiche z. B. EP 50 827). Über eine Wirksamkeit dieser vorbekannten Verbindungen als Schädlingsbekämpfungsmittel ist bisher nichts bekannt.

Es wurde gefunden, daß die teilweise bekannten substituierten Oxazolidinone der allgemeinen Formel (I),

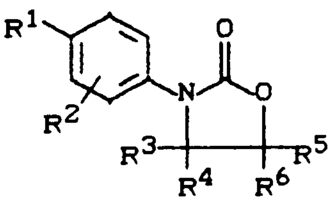

(I)

in welcher

| | |
|---|---|
| $R^1$ | für Trifluormethylthio, Trifluormethylsulfinyl, Trifluormethylsulfonyl oder für Fluorsulfonyl steht, |
| $R^2$ | für Wasserstoff, Cyano, Nitro, Halogen, Alkyl, Alkoxy, Halogenalkyl oder Halogenalkoxy steht, |
| $R^3$ und $R^5$ | unabhängig voneinander jeweils für Wasserstoff, Alkyl, Alkenyl, Alkinyl, Halogenalkyl oder für gegebenenfalls substituiertes Cycloalkyl stehen und |
| $R^4$ und $R^6$ | unabhängig voneinander jeweils für Wasserstoff oder Alkyl stehen, |

eine gute Wirksamkeit gegen Schädlinge besitzen.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die Verwendung der reinen Isomeren als auch die der Isomerengemische werden erfindungsgemäß beansprucht.

Überraschenderweise zeigen die erfindungsgemäß verwendbaren substituierten Oxazolidinone der allgemeinen Formel (I) eine erheblich bessere Wirksamkeit gegen pflanzenpathogene Pilze als die aus dem Stand der Technik bekannten Oxazolidinone, wie beispielsweise die Verbindung 5-Fluormethyl-3-(4-methylthiophenyl)-1,3-oxazolidin-2-on, welche chemisch und wirkungsmäßig naheliegende Verbindungen sind.

Die erfindungsgemäß verwendbaren substituierten Oxazolidinone sind durch die Formel (I) allgemein definiert. Bevorzugt verwendbar sind Verbindungen der Formel (I), bei welchen

| | |
|---|---|
| $R^1$ | für Trifluormethylthio, Trifluormethylsulfinyl, Trifluormethylsulfonyl oder für Fluorsulfonyl steht, |
| $R^2$ | für Wasserstoff, Cyano, Nitro, Fluor, Chlor, Brom, Iod, für jeweils geradkettiges oder verzweigtes Alkyl oder Alkoxy mit jeweils 1 bis 6 Kohlenstoffatomen oder für jeweils geradkettiges oder verzweigtes Halogenalkyl oder Halogenalkoxy mit jeweils 1 bis 6 Kohlenstoffatomen und jeweils 1 bis 13 gleichen oder verschiedenen Halogenatomen steht, |
| $R^3$ und $R^5$ | unabhängig voneinander jeweils für Wasserstoff, für jeweils geradkettiges oder verzweigtes Alkyl, Alkenyl oder Alkinyl mit jeweils bis zu 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für gegebenenfalls einfach oder mehrfach, gleich oder verschieden durch Halogen und/oder jeweils geradkettiges oder verzweigtes Alkyl und/oder Halogenalkyl mit jeweils 1 bis 4 Kohlenstoffatomen und gegebenenfalls 1 bis 9 gleichen oder verschiedenen Halogenatomen substituiertes Cycloalkyl mit 3 bis 7 Kohlenstoffatomen stehen und |

$R^4$ und $R^6$ unabhängig voneinander jeweils für Wasserstoff oder für geradkettiges oder verzweigtes Alkyl mit 1 bis 6 Kohlenstoffatomen stehen.

Besonders bevorzugt verwendbar sind Verbindungen der Formel (I), bei welchen

$R^1$ für Trifluormethylthio, Trifluormethylsulfinyl, Trifluormethylsulfonyl oder für Fluorsulfonyl steht,

$R^2$ für Wasserstoff, Cyano, Nitro, Fluor, Chlor, Brom, für jeweils geradkettiges oder verzweigtes Alkyl oder Alkoxy mit jeweils 1 bis 4 Kohlenstoffatomen oder für jeweils geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen steht,

$R^3$ und $R^5$ unabhängig voneinander jeweils für Wasserstoff, für jeweils geradkettiges oder verzweigtes Alkyl, Alkenyl oder Alkinyl mit jeweils bis zu 4 Kohlenstoffatomen, für geradkettiges oder verzweigtes Halogenalkyl mit 1 bis 4 Kohlenstoffatomen und 1 bis 9 gleichen oder verschiedenen Halogenatomen oder für gegebenenfalls ein- bis fünffach, gleich oder verschieden durch Halogen und/oder jeweils geradkettiges oder verzweigtes Alkyl und/oder Halogenalkyl mit jeweils 1 bis 3 Kohlenstoffatomen und gegebenenfalls 1 bis 7 gleichen oder verschiedenen Halogenatomen substituiertes Cycloalkyl mit 3 bis 6 Kohlenstoffatomen stehen und

$R^4$ und $R^6$ unabhängig voneinander jeweils für Wasserstoff oder für geradkettiges oder verzweigtes Alkyl mit 1 bis 4 Kohlenstoffatomen stehen.

Ganz besonders bevorzugt verwendbar sind Verbindungen der Formel (I), bei welchen

$R^1$ für Trifluormethylthio, Trifluormethylsulfinyl, Trifluormethylsulfonyl oder für Fluorsulfonyl steht,

$R^2$ für Wasserstoff, Cyano, Nitro, Fluor, Chlor, Brom, Methyl, Ethyl, n- oder i-Propyl, Methoxy, Ethoxy, n- oder i-Propoxy, Trifluormethyl, Difluormethyl, Trifluormethoxy oder Difluormethoxy steht,

$R^3$ und $R^5$ unabhängig voneinander jeweils für Wasserstoff, für Methyl, Ethyl, n- oder i-Propyl, n-, i-, s- oder t-Butyl, Vinyl, Allyl, Ethinyl, Propargyl, für Fluormethyl, Chlormethyl, Brommethyl, Iodmethyl, oder für gegebenenfalls einfach oder zweifach, gleich oder verschieden durch Fluor, Chlor, Brom, Methyl, Ethyl, n- oder i-Propyl, Difluormethyl oder Trifluormethyl substituiertes Cyclopropyl oder Cyclohexyl stehen und

$R^4$ und $R^6$ unabhängig voneinander jeweils für Wasserstoff, Methyl, Ethyl, n- oder i-Propyl stehen.

Im einzelnen sei auf die bei den Herstellungsbeispielen genannten Verbindungen verwiesen.

Die erfindungsgemäß verwendbaren substituierten Oxazolidinone der Formel (I) sind teilweise bekannt (vergleiche z. B. EP 50 827).

Noch nicht bekannt und ebenfalls Gegenstand der Erfindung sind substituierte Oxazolidinone der Formel (I-A),

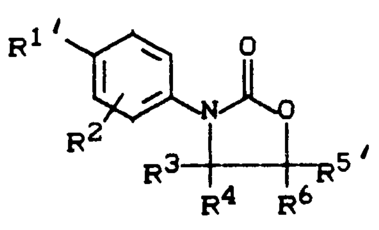

(I-A)

in welcher

$R^{1'}$ für Trifluormethylthio, Trifluormethylsulfinyl, Trifluormethylsulfonyl oder für Fluorsulfonyl steht,

$R^2$ für Wasserstoff, Cyano, Nitro, Halogen, Alkyl, Alkoxy, Halogenalkyl oder Halogenalkoxy steht,

$R^3$ und $R^{5'}$ unabhängig voneinander jeweils für Wasserstoff, Alkyl, Alkenyl, Alkinyl, Halogenalkyl oder für gegebenenfalls substituiertes Cycloalkyl stehen und

$R^4$ und $R^6$ unabhängig voneinander jeweils für Wasserstoff oder Alkyl stehen,

ausgenommen die Verbindungen 5-Chlormethyl-3-(4-trifluormethylthiophenyl)- und 5-Chlormethyl-3-(4-trifluormethylsulfonylphenyl)-1,3-oxazolidin-2-on.

Man erhält bekannte und neue substituierte Oxazolidinone dar Formel (I), wenn man

(a) substituierte Isocyanate der Formel (II),

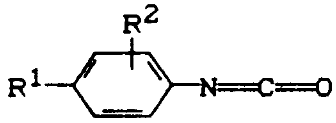

(II)

in welcher

$R^1$ (welches $R^{1'}$ beinhaltet) und $R^2$ die oben angegebene Bedeutung haben,

mit Oxiranen der Formel (III),

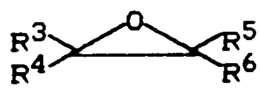

(III)

in welcher

$R^3$, $R^4$, $R^5$ (welches $R^{5'}$ beinhaltet) und $R^6$ die oben angegebene Bedeutung haben,

gegebenenfalls in Gegenwart eines Verdünnungsmittels und gegebenenfalls in Gegenwart eines Reaktionshilfsmittels umsetzt oder wenn man

(b) die mit Hilfe des Herstellungsverfahrens (a) erhältlichen substituierten Oxazolidinone der Formel (Ia),

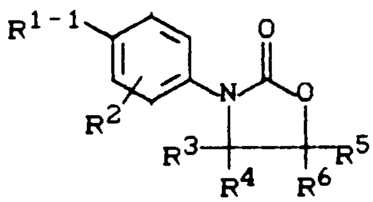

(Ia)

in welcher

$R^{1-1}$ für Trifluormethylthio steht und

$R^2$, $R^3$, $R^4$, $R^5$ und $R^6$ die oben angegebene Bedeutung haben,

mit einem Oxidationsmittel gegebenenfalls in Gegenwart eines Verdünnungsmittels und gegebenenfalls in Gegenwart eines Reaktionshilfsmittels umsetzt.

Verwendet man beispielsweise 4-Trifluormethylthiophenyl-isocyanat und Epibromhydrin als Ausgangsstoffe, so läßt sich der Reaktionsablauf des Herstellungsverfahrens (a) durch das folgende Formelschema darstellen:

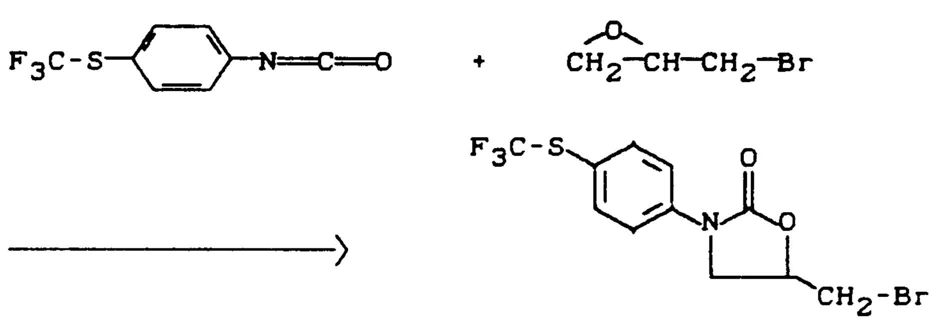

Verwendet man beispielsweise 5-Brommethyl-3-(4-trifluormethylthio-phenyl)-1,3-oxazolidin-2-on als Ausgangsverbindung und Wasserstoffperoxid als Oxidationsmittel, so läßt sich der Reaktionsablauf des Herstellungsverfahrens (b) durch das folgende Formelschema darstellen:

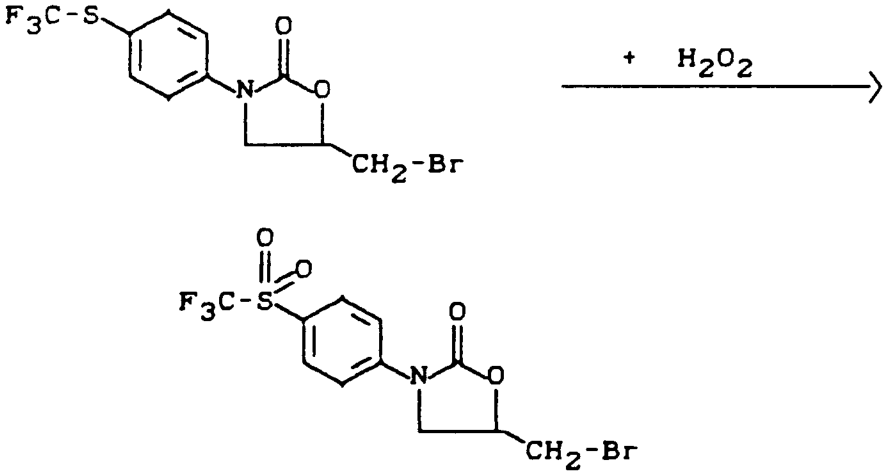


Die zur Durchführung des Herstellungsverfahrens (a) als Ausgangsstoffe benötigten Isocyanate sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäß verwendbaren Verbindungen der Formel (I) als bevorzugt für diese Substituenten genannt wurden.

Die Isocyanate der Formel (II) sind bekannt oder erhältlich in Analogie zu bekannten Verfahren (vergleiche z. B. EP 72 528; EP 50 827; DE 29 17 618; DE 23 34 355; ZA 68 04 506 [1968]; DE 879 550 [1939]).

Die zur Durchführung des Herstellungsverfahrens (a) weiterhin als Ausgangsstoffe benötigten Oxirane sind durch die Formel (III) allgemein definiert. In dieser Formel (III) stehen $R^3$, $R^4$, $R^5$ und $R^6$ vorzugsweise für diejenigen Reste, die bereits im Zusammenhang mit der Beschreibung der erfindungsgemäß verwendbaren Verbindungen der Formel (I) als bevorzugt für diese Substituenten genannt wurden.

Die Oxirane der Formel (III) sind allgemein bekannt oder erhältlich in Analogie zu bekannten Verfahren (vergleiche z. B. EP 368 656; EP 287 347; US 4.587.217; EP 91 305; J. Org. Chem. 45, 3930-3932 [1980]; EP 275 221).

$R^{1'}$ und $R^{5'}$ haben die Bedeutungen von $R^1$ und $R^5$, die bereits in der Beschreibung gegeben wurden.

Die zur Durchführung des Herstellungsverfahrens (b) als Ausgangsstoffe benötigten substituierten Oxazolidinone sind durch die Formel (Ia) allgemein definiert. In dieser Formel (Ia) stehen $R^2$, $R^3$, $R^4$, $R^5$ und $R^6$ vorzugsweise für diejenigen Reste, die bereits im Zusammenhang mit der Beschreibung der erfindungsgemäß verwendbaren Verbindungen der Formel (I) als bevorzugt für diese Substituenten genannt wurden.
$R^{1-1}$ steht vorzugsweise für einen Trifluormethylthiorest.
Die substituierten Oxazolidinone sind erfindungsgemäß verwendbare Verbindungen und erhältlich mit Hilfe des Herstellungsverfahrens (a).

Als Verdünnungsmittel zur Durchführung des Herstellungsverfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether wie Diethylether, Diisopropylether, Dioxan, Tetrahydrofuran oder Ethylenglykoldimethyl- oder -diethylether, Ketone wie Aceton, Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigssäureethylester oder Sulfoxide wie Dimethylsulfoxid.

Das Herstellungsverfahren (a) wird vorzugsweise in Gegenwart eines geeigneten Reaktionshilfsmittels durchgeführt. Als solche kommen insbesondere Alkalimetallhalogenide infrage. Mit besonderem Vorzug verwendet man Lithiumhalogenide wie beispielsweise Lithiumchlorid oder Lithiumbromid.

Die Reaktionstemperaturen können bei der Durchführung des Herstellungsverfahrens (a) in einem größeren Bereich variiert werden. Im allgemeinen arbeitet man bei Temperaturen zwischen 50 °C und 150 °C, vorzugsweise bei Temperaturen zwischen 80 °C und 120 °C.

Zur Durchführung des Herstellungsverfahrens (a) setzt man pro Mol an Isocyanat der Formel (II) im allgemeinen 0.7 bis 2.0 Mol, vorzugsweise 0.9 bis 1.25 Mol an Oxiran der Formel (III) und gegebenenfalls 0.001 bis 2.0 Mol, vorzugsweise 0.01 bis 1.0 Mol an Reaktionshilfsmittel ein.
Die Reaktionsdurchführung, Aufarbeitung und Isolierung der Reaktionsprodukte erfolgt nach bekannten

Verfahren (vergleiche hierzu beispielsweise US 4.128.654 oder die Herstellungsbeispiele).

Als Oxidationsmittel zur Durchführung des Herstellungsverfahrens (b) kommen alle üblicherweise für Schwefeloxidationen verwendbaren Oxidationsmittel infrage. Vorzugsweise verwendet man Wasserstoffperoxid oder organische Persäuren, wie beispielsweise Peressigsäure, 4-Nitroperbenzoesäure oder 3-Chlorperbenzoesäure oder auch anorganische Oxidationsmittel, wie Periodsäure, Kaliumpermanganat oder Chromsäure.

Als Verdünnungsmittel zur Durchführung des Herstellungsverfahrens (b) kommen in Abhängigkeit vom verwendeten Oxidationsmittel anorganische oder organische Lösungsmittel infrage. Vorzugsweise verwendet man Alkohole wie beispielsweise Methanol oder Ethanol oder deren Gemische mit Wasser oder reines Wasser; Säuren wie beispielsweise Essigsäure, Acetanhydrid oder Propionsäure oder dipolar aprotische Lösungsmittel wie Acetonitril, Aceton, Essigsäureethylester oder Dimethylformamid sowie auch gegebenenfalls halogenierte Kohlenwasserstoffe wie Benzin, Benzol, Toluol, Hexan, Cyclohexan, Petrolether, Dichlormethan, Dichlorethan, Chloroform, Tetrachlorkohlenstoff oder Chlorbenzol.

Das Herstellungsverfahren (b) kann gegebenenfalls in Gegenwart eines Reaktionshilfsmittels durchgeführt werden. Als solche kommen alle üblicherweise verwendbaren organischen oder anorganischen Säurebindemittel infrage. Vorzugsweise verwendet man Erdalkalimetall- oder Alkalimetallhydroxide, -acetate oder -carbonate, wie beispielsweise Calciumhydroxid, Natriumhydroxid, Natriumacetat oder Natriumcarbonat.

Das Herstellungsverfahren (b) kann gegebenenfalls auch in Gegenwart eines geeigneten Katalysators durchgeführt werden. Als solche kommen alle üblicherweise für derartige Schwefeloxidationen verwendbaren Katalysatoren infrage. Vorzugsweise verwendet man Schwermetallkatalysatoren; beispielhaft genannt sei in diesem Zusammenhang Ammoniummolybdat.

Die Reaktionstemperaturen können bei der Durchführung des Herstellungsverfahrens (b) in einem größeren Bereich variiert werden. Im allgemeinen arbeitet man bei Temperaturen zwischen -30 °C und +100 °C, vorzugsweise bei Temperaturen zwischen 0 °C und +80 °C.

Zur Durchführung des Herstellungsverfahrens (b) setzt man pro Mol an substituierten Oxazolidinon der Formel (Ia) im allgemeinen 2.0 bis 10.0 Mol, vorzugsweise 2.0 bis 5.0 Mol an Oxidationsmittel, gegebenenfalls 1.0 bis 1.5 Mol, vorzugsweise 1.0 bis 1.3 Mol an als Reaktionshilfsmittel verwendeter Base und gegebenenfalls 0.001 bis 1.0 Mol, vorzugsweise 0.005 bis 0.05 Mol an Katalysator ein.
Die Reaktionsdurchführung, Aufarbeitung und Isolierung der Reaktionsprodukte erfolgt nach bekannten Verfahren (vergleiche hierzu die Herstellungsbeispiele).

Die erfindungsgemäß verwendbaren Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;

Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäß verwendbaren Wirkstoffe mit besonders gutem Erfolg zur Bekämpfung von Getreidekrankheiten, wie beispielsweise gegen den Erreger der Netzfleckenkrankheit der Gerste (Pyrenophora teres) oder gegen den Erreger des echten Getreidemehltaues (Erysiphe graminis) oder gegen den Erreger der Braunfleckenkrankheit an Gerste oder Weizen (Cochliobolus sativus) oder gegen den Erreger der Braunspelzigkeit des Weizens (Leptosphaeria nodorum) oder gegen den Erreger des Getreideschneeschimmels (Fusarium nivale) oder zur Bekämpfung von Krankheiten im Obst- und Gemüseanbau, wie beispielsweise gegen den Erreger der Tomatenbraunfäule (Phytophthora infestans) oder zur Bekämpfung von Reiskrankheiten, wie beispielsweise gegen den Erreger der Reisfleckenkrankheit (Pyricularia oryzae) einsetzen. Dabei zeigen die erfindungsgemäß verwendbaren Wirkstoffe neben protektiver Wirksamkeit auch kurative Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können in den Formulierungen in Mischung mit anderen bekannten Wirkstoffen vorliegen wie Fungizide, Insektizide, Akarizide und Herbizide sowie in Mischungen

mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-% am Wirkungsort erforderlich.

## Herstellungsbeispiele:

### Beispiel 1

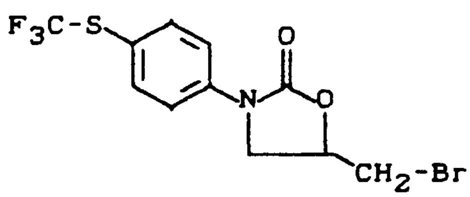

(Verfahren a)

Zu 6,9 g (0,05 Mol) Epibromhydrin in 60 ml Dimethylformamid gibt man nacheinander 1 g (0,035 Mol) Lithiumchlorid und 12 g (0,055 Mol) 4-Trifluormethylphenylisocyanat (vergleiche z. B. EP 50 827) und rührt anschließend 48 Stunden bei 140 °C. Zur Aufarbeitung gibt man den abgekühlten Reaktionsansatz in Wasser, extrahiert mit Dichlormethan, wäscht die organische Phase zweimal mit Wasser, trocknet über Natriumsulfat und engt im Vakuum ein. Der Rückstand wird durch Chromatographie an Kieselgel (Laufmittel: Diethylether) gereinigt.

Man erhält 3,0 g (17 % der Theorie) an 5-Brommethyl-3-(4-trifluormethylthiophenyl)-1,3-oxazolidin-2-on vom Schmelzpunkt 75 °C.

$^1H$-NMR ($CDCl_3$/Tetramethylsilan) $\delta$ = 3,800; 3,798 ppm.

### Beispiel 2

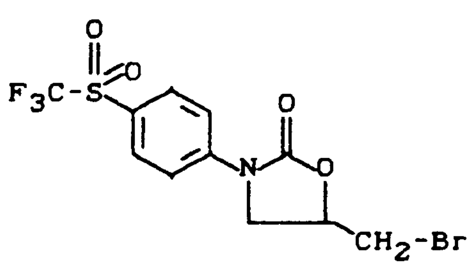

(Verfahren b)

2 g (0,0056 Mol) 5-Brommethyl-3-(4-trifluormethylthiophenyl)-1,3-oxazolidin-2-on und 2,2 g (0,023 Mol) 35 %-iges Wasserstoffperoxid werden in 10 ml Eisessig 7 Stunden bei 50 °C gerührt; anschließend wird der

Reaktionsansatz in Wasser gegeben, der Überstand abdekantiert, der Rückstand in Essigester gelöst, mit wässriger Natriumhydrogencarbonatlösung gewaschen, die organische Phase über Natriumsulfat getrocknet und im Vakuum eingeengt. Der Rückstand kann durch Chromatographie an Kieselgel (Laufmittel: Essigester / Cyclohexan 3:1) gereinigt werden.

Man erhält 1,8 g (83 % der Theorie) an 5-Brommethyl-3-(4-trifluormethylsulfonylphenyl)-1,3-oxazolidin-2-on als Öl.

$^1$H-NMR ($CDCl_3$/Tetramethylsilan) $\delta$ = 3,840; 3,824 ppm.

In entsprechender Weise und gemäß den allgemeinen Angaben zur Herstellung erhält man die folgenden substituierten Oxazolidinone der allgemeinen Formel (I):

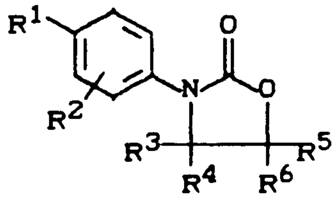

(I)

| Bsp. Nr. | $R^1$ | $R^2$ | $R^3$ | $R^4$ | $R^5$ | $R^6$ | physikalische Eigenschaften |
|---|---|---|---|---|---|---|---|
| 3 | $-SCF_3$ | H | H | H | $-CH_2-F$ | H | Fp. 116° C |
| 4 | $-SO_2-CF_3$ | H | H | H | $-CH_2-F$ | H | Fp. 82° C |
| 5 | $-SCF_3$ | H | H | H | $-CH_2-Cl$ | H | Fp. 80° C |
| 6 | $-SCF_3$ | H | H | H | $-CH_2-I$ | H | $^1$H-NMR*): 3,800; 3,789 |
| 7 | $-SO_2-CF_3$ | H | H | H | $-CH_2-Cl$ | H | Fp. 97° C |
| 8 | $-SCF_3$ | H | H | H | $-CH_2-F$ | $CH_3$ | Fp. 86° C |
| 9 | $-SO_2-CF_3$ | H | H | H | $-CH_2-F$ | $CH_3$ | Fp. 94° C |
| 10 | $-SO_2-F$ | H | H | H | $-CH_2-F$ | H | Fp. 123° C |
| 11 | $-SO_2-F$ | H | H | H | $-CH_2-Cl$ | H | Fp. 50° C |
| 12 | $-SO_2-F$ | H | H | H | $-CH_2-Br$ | H | $^1$H-NMR*): 3,84; 3,85 |
| 13 | $-SO_2-F$ | H | H | H | $-CH_2-F$ | $CH_3$ | $^1$H-NMR*): 1,59; 1,60 |
| 14 | $-SO_2-F$ | H | H | H | $-C_2H_5$ | H | $^1$H-NMR*): 1,085 |

| Bsp. Nr. | $R^1$ | $R^2$ | $R^3$ | $R^4$ | $R^5$ | $R^6$ | physikalische Eigenschaften |
|---|---|---|---|---|---|---|---|
| 15 | $-SO_2-F$ | H | H | H | $-CH_2-I$ | H | $^1H$-NMR*): 3,83; 3,84 |
| 16 | $-SO_2-F$ | H | $-CH_2-Cl$ | H | $-CH_2-Cl$ | H | Fp. 125° C |
| 17 | $-SO_2F$ | H | H | H | $-CH=CH_2$ | H | Fp. 110° C |
| 18 | $-SO_2CF_3$ | H | H | H | $-CH=CH_2$ | H | Fp. 127° C |
| 19 | $-SO_2-CF_3$ | H | H | $-CH=CH_2$ | H | H | Fp. 71-72° C |
| 20 | $-SO_2-F$ | H | $-CH_2-Cl$ | H | $-CH_2-Cl$ | H | Fp. 79-81° C |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurde die nachstehende aufgeführte Verbindung als Vergleichssubstanz eingesetzt:

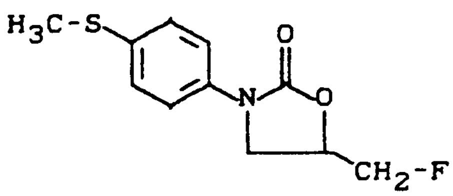

(A)

5-Fluormethyl-3-(4-methylthiophenyl)-1,3-oxazolidin-2-on
(vergleiche z. B. US 4.128.654).

Beispiel A

Phytophthora-Test (Tomate) / protektiv und kurativ

Lösungsmittel: 4,7 Gewichtsteile Aceton
Emulgator: 0,3 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

A1: Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

A2: Zur Prüfung auf kurative Wirksamkeit werden junge Pflanzen mit einer wäßrigen Sporensuspension von Phytophthora infestans inokuliert und verbleiben 7 Stunden bei 20°C und 100 % relativer Luftfeuchtigkeit in einer Inkubationskabine. Nach einer kurzen Antrocknungszeit werden die Pflanzen mit der Wirkstoffzubereitung tropfnaß gespritzt.

Die Pflanzen werden dann in einer Inkubationskabine bei 20°C und einer relativen Luftfeuchtigkeit von ca. 100 % aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen: 2 und 7.

## Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß den Herstellungsbeispielen: 3, 5 und 11.

## Beispiel C

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß den Herstellungsbeispielen: 2 und 11.

## Patentansprüche

**1.** Verwendung von substituierten Oxazolidinonen der allgemeinen Formel (I),

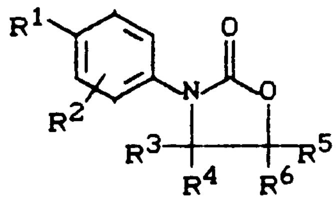

(I)

in welcher

- $R^1$ für Trifluormethylthio, Trifluormethylsulfinyl, Trifluormethylsulfonyl oder für Fluorsulfonyl steht,
- $R^2$ für Wasserstoff, Cyano, Nitro, Halogen, Alkyl, Alkoxy, Halogenalkyl oder Halogenalkoxy steht,
- $R^3$ und $R^5$ unabhängig voneinander jeweils für Wasserstoff, Alkyl, Alkenyl, Alkinyl, Halogenalkyl oder für gegebenenfalls substituiertes Cycloalkyl stehen und
- $R^4$ und $R^6$ unabhängig voneinander jeweils für Wasserstoff oder Alkyl stehen,

deren geometrische und optischen Isomere und Isomerengemische zur Bekämpfung von Schädlingen.

**2.** Verwendung von Verbindungen der Formel (I) gemäß Anspruch 1, in welcher

- $R^1$ für Trifluormethylthio, Trifluormethylsulfinyl, Trifluormethylsulfonyl oder für Fluorsulfonyl steht,
- $R^2$ für Wasserstoff, Cyano, Nitro, Fluor, Chlor, Brom, Iod, für jeweils geradkettiges oder verzweigtes Alkyl oder Alkoxy mit jeweils 1 bis 6 Kohlenstoffatomen oder für jeweils geradkettiges oder verzweigtes Halogenalkyl oder Halogenalkoxy mit jeweils 1 bis 6 Kohlenstoffatomen und jeweils 1 bis 13 gleichen oder verschiedenen Halogenatomen steht,
- $R^3$ und $R^5$ unabhängig voneinander jeweils für Wasserstoff, für jeweils geradkettiges oder verzweigtes Alkyl, Alkenyl oder Alkinyl mit jeweils bis zu 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für gegebenenfalls einfach oder mehrfach, gleich oder verschieden durch Halogen und/oder jeweils geradkettiges oder verzweigtes Alkyl und/oder Halogenalkyl mit jeweils 1 bis 4 Kohlenstoffatomen und gegebenenfalls 1 bis 9 gleichen oder verschiedenen Halogenatomen substituiertes Cycloalkyl mit 3 bis 7 Kohlenstoffatomen stehen und
- $R^4$ und $R^6$ unabhängig voneinander jeweils für Wasserstoff oder für geradkettiges oder verzweigtes Alkyl mit 1 bis 6 Kohlenstoffatomen stehen.

**3.** Verwendung der Verbindungen der Formel (I) gemäß Anspruch 1, in welcher

- $R^1$ für Trifluormethylthio, Trifluormethylsulfinyl, Trifluormethylsulfonyl oder für Fluorsulfonyl steht,
- $R^2$ für Wasserstoff, Cyano, Nitro, Fluor, Chlor, Brom, für jeweils geradkettiges oder verzweigtes Alkyl oder Alkoxy mit jeweils 1 bis 4 Kohlenstoffatomen oder für jeweils geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen steht,
- $R^3$ und $R^5$ unabhängig voneinander jeweils für Wasserstoff, für jeweils geradkettiges oder verzweigtes Alkyl, Alkenyl oder Alkinyl mit jeweils bis zu 4 Kohlenstoffatomen, für geradkettiges oder verzweigtes Halogenalkyl mit 1 bis 4 Kohlenstoffatomen und 1 bis 9 gleichen oder verschiedenen Halogenatomen oder für gegebenenfalls ein- bis fünffach, gleich oder verschieden durch Halogen und/oder jeweils geradkettiges oder verzweigtes Alkyl und/oder Halogenalkyl mit jeweils 1 bis 3 Kohlenstoffatomen und gegebenenfalls 1 bis 7 gleichen oder verschiedenen Halogenatomen substituiertes Cycloalkyl mit 3 bis 6 Kohlenstoffatomen stehen und
- $R^4$ und $R^6$ unabhängig voneinander jeweils für Wasserstoff oder für geradkettiges oder verzweigtes Alkyl mit 1 bis 4 Kohlenstoffatomen stehen.

**4.** Verwendung von Verbindungen der Formel (I) gemäß Anspruch 1, in welcher

$R^1$ für Trifluormethylthio, Trifluormethylsulfinyl, Trifluormethylsulfonyl oder für Fluorsulfonyl steht,

$R^2$ für Wasserstoff, Cyano, Nitro, Fluor, Chlor, Brom, Methyl, Ethyl, n- oder i-Propyl, Methoxy, Ethoxy, n- oder i-Propoxy, Trifluormethyl, Difluormethyl, Trifluormethoxy oder Dilfuormethoxy steht,

$R^3$ und $R^5$ unabhängig voneinander jeweils für Wasserstoff, für Methyl, Ethyl, n- oder i-Propyl, n-, i-, s- oder t-Butyl, Vinyl, Allyl, Ethinyl, Propargyl, für Fluormethyl, Chlormethyl, Brommethyl, Iodmethyl, oder für gegebenenfalls einfach oder zweifach, gleich oder verschieden durch Fluor, Chlor, Brom, Methyl, Ethyl, n- oder i-Propyl, Difluormethyl oder Trifluormethyl substituiertes Cyclopropyl oder Cyclohexyl stehen und

$R^4$ und $R^6$ unabhängig voneinander jeweils für Wasserstoff, Methyl, Ethyl, n- oder i-Propyl stehen.

**5.** Schädlingsbekämpfungsmittel, gekennzeichnet durch einen Gehalt an mindestens einem substituierten Oxazolidinon der Formel (I) nach den Ansprüchen 1 bis 4.

**6.** Verfahren zur Bekämpfung von Schädlingen, dadurch gekennzeichnet, daß man mindestens ein substituiertes Oxazolidinon der Formel (I) nach den Ansprüchen 1 bis 4 auf Schädlinge und/oder ihren Lebensraum einwirken läßt.

**7.** Verfahren zur Herstellung von Schädlingsbekämpfungsmitteln, dadurch gekennzeichnet, daß man substituierte Oxazolidinone der Formel (I) nach den Ansprüchen 1 bis 4 mit Streckmitteln und/oder oberflächenaktiven Mitteln vermischt.

**8.** Substituierte Oxazolidinone der allgemeinen Formel (I-A)

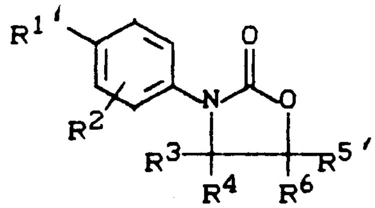

(I-A)

in welcher

$R^{1'}$ für Trifluormethylthio, Trifluormethylsulfinyl, Trifluormethylsulfonyl oder für Fluorsulfonyl steht,

$R^2$ für Wasserstoff, Cyano, Nitro, Halogen, Alkyl, Alkoxy, Halogenalkyl oder Halogenalkoxy steht,

$R^3$ und $R^{5'}$ unabhängig voneinander jeweils für Wasserstoff, Alkyl, Alkenyl, Alkinyl, Halogenalkyl oder für gegebenenfalls substituiertes Cycloalkyl stehen und

$R^4$ und $R^6$ unabhängig voneinander jeweils für Wasserstoff oder Alkyl stehen,

deren geometrische und optischen Isomere und Isomerengemische, ausgenommen die Verbindungen 5-Chlormethyl-3-(4-trifluormethylthiophenyl)- und 5-Chlormethyl-3-(4-trifluormethylsulfonylphenyl)-1,3-oxazolidin-2-on.

**9.** Verfahren zur Herstellung von substituierten Oxazolidinonen der allgemeinen Formel (I-A)

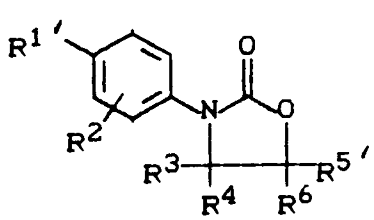

(I-A)

in welcher

$R^{1'}$ für Trifluormethylthio, Trifluormethylsulfinyl, Trifluormethylsulfonyl oder für Fluorsulfonyl steht,

$R^2$ für Wasserstoff, Cyano, Nitro, Halogen, Alkyl, Alkoxy, Halogenalkyl oder Halogenalkoxy steht,

$R^3$ und $R^{5'}$ unabhängig voneinander jeweils für Wasserstoff, Alkyl, Alkenyl, Alkinyl, Halogenalkyl oder für gegebenenfalls substituiertes Cycloalkyl stehen und

$R^4$ und $R^6$ unabhängig voneinander jeweils für Wasserstoff oder Alkyl stehen, ausgenommen die Verbindungen 5-Chlormethyl-3-(4-trifluormethylthiophenyl)- und 5-Chlormethyl-3-(4-trifluormethylsulfonylphenyl)-1,3-oxazolidin-2-on,

dadurch gekennzeichnet, daß man

(a) substituierte Isocyanate der Formel (II),

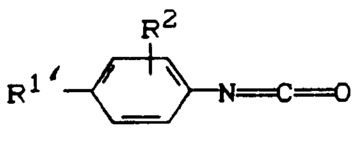

(II)

in welcher

$R^{1'}$ und $R^2$ die oben angegebene Bedeutung haben,

mit Oxiranen der Formel (III),

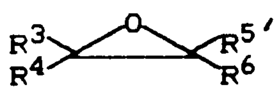

(III)

in welcher

$R^3$, $R^4$, $R^{5'}$ und $R^6$ die oben angegebene Bedeutung haben,

gegebenenfalls in Gegenwart eines Verdünnungsmittel und gegebenenfalls in Gegenwart eines Reaktionshilfsmittels umsetzt oder wenn man

(b) die mit Hilfe des Herstellungsverfahrens (a) erhältlichen substituierten Oxazolidinone der Formel (Ia),

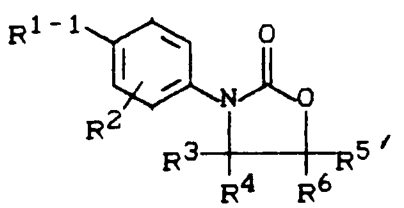

(Ia)

in welcher

$R^{1-1}$ für Trifluormethylthio steht und

$R^2$, $R^3$, $R^4$, $R^{5'}$ und $R^6$ die oben angegebene Bedeutung haben,

mit einem Oxidationsmittel gegebenenfalls in Gegenwart eines Verdünnungsmittels und gegebenenfalls in Gegenwart eines Reaktionshilfsmittels umsetzt.

Europäisches Patentamt

# EUROPÄISCHER RECHERCHENBERICHT

Nummer der Anmeldung

EP 92 10 9193

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | US-A-4 128 654 (ROBERT B. FUGITT ET AL) * das ganze Dokument * | 1-8 | C07D263/20 A01N43/76 |
| D,Y | EP-A-0 050 827 (E.I. DU PONT DE NEMOURS AND COMPANY) * Ansprüche * | 1-8 | |

RECHERCHIERTE SACHGEBIETE (Int. Cl.5)

C07D
A01N

Der vorliegende Recherchenbericht wurde für alle Patentansprüche erstellt

| Recherchenort | Abschlußdatum der Recherche | Prüfer |
|---|---|---|
| DEN HAAG | 02 SEPTEMBER 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)